# EUROPEAN PATENT APPLICATION

(11) **EP 4 190 898 A1**
(43) Date of publication of application: **07.06.2023**
(21) Application number: 20947006.1
(22) Date of filing: 28.07.2020
(51) Int. Cl.: C12N 9/38, C12R 1/09

(54) **METHOD FOR PREPARING BETA-GALACTOSIDASE AND APPLICATION THEREOF**

(71) Applicant: Quantum Hi-Tech (Guangdong) Biological Co., Ltd., Guangdong 529081 (CN)
(72) Inventor: WEI, Yuan'an, Jiangmen, Guangdong 529000 (CN); ZENG, Xianwei, Jiangmen, Guangdong 529000 (CN); XIE, Zhilong, Jiangmen, Guangdong 529000 (CN); YANG, Xinqiu, Jiangmen, Guangdong 529000 (CN); CHEN, Zijian, Jiangmen, Guangdong 529000 (CN); HUAN, Jingang, Jiangmen, Guangdong 529000 (CN)
(74) Representative: Marks & Clerk LLP
(86) International application number: PCT/CN2020/105159
(87) International publication number: WO 2022/021057

(57) **Abstract**

The present disclosure belongs to the technical field of biochemical engineering, and relates to a preparation method of β-galactosidase. The preparation method includes the following steps: step 1, seeding *Bacillus circulans* into a Luria-Bertani (LB) medium for culture and activation and then into a seed tank for culture to obtain a seed culture broth; step 2, introducing the seed culture broth into a fermentor containing a fermentation medium for fermentation to obtain a fermentation broth of *B. circulans;* and step 3, filtering or centrifuging the fermentation broth of *B. circulans* to remove cells to obtain a β-galactosidase liquid; the fermentation medium includes lactose, galactose, phytone, corn meal, yeast extract, a phosphate salt, a carbonate salt, and water. According to the method, fermentation time is short, production period is shortened, and specific activity of the resulting enzyme liquid is high. The present disclosure further provides a preparation method of an immobilized β-galactosidase without using a crosslinking agent. The resulting immobilized enzyme has excellent stability and can be continuously used for 264 hours or more, and costs for preparing galactose by using the immobilized enzyme is substantially reduced.

## Description

### TECHNICAL FIELD

The present disclosure belongs to the technical field of biochemical engineering, and particularly relates to a preparation method of β-galactosidase and use thereof.

### BACKGROUND

Galacto-oligosaccharides (GOSs) are a class of oligosaccharides. Its molecular structure is generally a galactose or glucose molecule with 1-7 galactosyl groups attached. Individual GOS components are present in infinitesimal amounts in animal milk, and trace amounts in breast milk. GOSs have high stability, even under acidic conditions. GOSs have the following characteristics: they are not digestible by human digestive enzymes; they are rarely absorbed by the human small intestine, they have excellent bifidobacterium proliferative activity, they inhibit the growth of harmful pathogenic and spoilage bacteria, and they promote the absorption of calcium, magnesium, and potassium. In addition, because they possess similar properties to water-soluble dietary fiber, GOSs can encourage bowel movements by drawing water into the bowel. Therefore, GOSs are widely used in the food industry. For example, GOSs are used as nutritional fortifiers and added to infant formula milk powder.

GOSs are prepared from the catalysis of lactose (food-grade lactose, pharmaceutical-grade lactose, organic lactose, filtered whey powder, whey liquid, and the like) with β-galactosidase (EC 3.2.1.23). In nature, β-galactosidase is widely present; it can be from fruits, such as apples and apricots, or microorganisms, such as bacteria, yeasts, and molds. Due to the high yield, short production cycle, and low production cost of β-galactosidase derived from microorganisms, microorganisms are often used as raw materials in the large-scale production of β-galactosidase. GOSs are usually produced by: (1) allowing free cells and an enzyme liquid to directly act on lactose to obtain GOSs; or (2) symbiotically fermenting lactose and microorganisms to obtain GOSs. However, one of the disadvantages of these methods is that impurities, such as free cells or proteins and even secondary metabolites, are introduced, leading to a need for complicated separation and purification processes; another disadvantage of these methods is that the utilization of enzymes is not high, which is not conducive to cost control.

In order to solve the aforementioned problems, the enzyme production performance of β-galactosidase and the method of using β-galactosidase need to be optimized. An optimization method present in large-scale production is enzyme immobilization to reuse the enzyme. However, in the process of enzyme immobilization, it is often necessary to use glutaraldehyde as a crosslinking agent for an immobilized enzyme carrier, but the use of crosslinking agents is sometimes restricted. In addition, in the prior art, immobilized β-galactosidase can only be recycled 8 times (patent application No. 201480049114.3), and the utilization rate of the enzyme is not high. Moreover, in the prior art, production processes of β-galactosidase directly by microbial fermentation have shortcomings of long fermentation period and complicated fermentation process.

Therefore, it is desired to provide a new preparation method of an immobilized β-galactosidase. The immobilized enzyme prepared by this method should have excellent stability and can be used in a plurality of cycles. The immobilized enzyme should also be able to improve the utilization rate of the enzyme. In addition, it is also necessary to provide a new preparation method of β-galactosidase, which can shorten the fermentation period and simplify the fermentation process. The immobilized enzyme prepared from the enzyme liquid produced by the fermentation process has more desirable properties.

### SUMMARY

The present disclosure aims to solve at least one of the above technical problems existing in the prior art. Thus, an objective of the present disclosure is to provide a preparation method of β-galactosidase. The method produces β-galactosidase using *Bacillus circulans.* The method simplifies the fermentation process and shortens the fermentation and production cycle.

Another objective of the present disclosure is to provide a preparation method of an immobilized β-galactosidase. The method does not require the use of a crosslinking agent, thus prevents any potential impact on food safety due to the presence of residual crosslinking agent; the immobilized enzyme prepared has excellent stability and can be used in more than 10 cycles.

Still another objective of the present disclosure is to provide a preparation method of GOSs. One feature of this method is that the immobilized β-galactosidase prepared by the present disclosure catalyzes the lactose reaction to produce the GOSs.

A preparation method of β-galactosidase is provided, including the following steps:
step 1, seeding *B. circulans* into a Luria-Bertani (LB) medium for culture and activation and then into a seed tank for culture to obtain a seed culture broth;
step 2, introducing the seed culture broth obtained in step 1 into a fermentor containing a fermentation medium for fermentation to obtain a fermentation broth of *B. circulans*; and
step 3, filtering or centrifuging the fermentation broth of *B. circulans* obtained in step 2 to remove cells to obtain a β-galactosidase liquid;
where the fermentation medium is supplemented with lactose, galactose, phytone, corn meal, yeast extract, a phosphate salt, a carbonate salt, and water.

Preferably, the *B. circulans* used in step 1 may be *B. circulans* purchased from American Type Culture Collection (ATCC), accession number: 31382; more preferably, the *B. circulans* used in step 1 may be *B. circulans* CCTCC NO: M 2015424, deposited in the China Center for Type Culture Collection (CCTCC). The *B. circulans* CCTCC NO: M 2015424 is induced from *B. circulans* ATCC No. 31382 by ultraviolet ray and lithium chloride.

Preferably, the LB medium in step 1 may include 8-15 g/L peptone, 2-8 g/L yeast extract, and 2-8 g/L of sodium chloride; more preferably, the LB medium in step 1 may include 10 g/L of peptone, 5 g/L of yeast extract, and 5 g/L of sodium chloride.

Preferably, the culture in step 1 may be conducted at 36-38°C for 16-24 h.

Preferably, the culture after seeding into the seed tank in step 1 may be conducted at 36-38°C for 5-7 h.

Preferably, after the culture and activation in step 1, steps before the seeding may include: introducing cultured and activated *B. circulans* to 20-500 mL of LB medium for shaking culture for 5-8 h, followed by seeding.

Preferably, the fermentation medium in step 2 may include 5-30 g/L lactose, 5-10 g/L galactose, 10-30 g/L phytone, 2-5g/L dried corn steep liquor powder, 2-4 g/L yeast extract, 2-4 g/L the phosphate salt, 1-2 g/L the carbonate salt, the balance being water.

Preferably, the fermentation medium may further include a defoamer with a content of 0.5-2 g/L.

Preferably, the fermentation in step 2 may be conducted at a pH of 5.5-8.0 for 24-35 h24-35 h.

Preferably, the fermentation in step 2 may be conducted at 36-38°C, pH 6.0-7.6, aeration (air) rate of 80-160 L/min, and stirring speed of 120-220 rpm.

More preferably, the fermentation in step 2 may be conducted at a pH of 6.5-7.0 with 0-8 h and pH 7.2-7.6 after 8 h, and still more preferably, pH 7.3-7.5 after 8 h.

Preferably, the filtration in step 3 may be microfiltration or ceramic membrane filtration. Microfiltration removes cells in the fermentation broth while reducing the introduction of contaminative microbes, ensuring that the enzyme liquid is clean and hygienic.

Preferably, the filtration in step 3 may be conducted by using a microfiltration membrane with a pore size of 0.2-0.8 µm.

Preferably, the centrifugation in step 3 is conducted by a disc centrifuge.

Preferably, after the β-galactosidase liquid is obtained in step 3, concentration treatment may further be included. The concentration treatment includes using 10-35% (mass fraction) of ammonium sulfate or sodium chloride to salt out and purify the resulting β-galactosidase liquid, followed by concentrating with a 10-100 KD membrane. This treatment may ensure that the β-galactosidase liquid has a conductivity of 4-20 ms/cm and an enzyme activity of 80-120 U/mL.

Preferably, the corn meal may be a dried corn steep liquor powder.

Preferably, the phosphate salt may be at least one selected from the group consisting of diammonium hydrogen phosphate, ammonium dihydrogen phosphate, calcium hydrogen phosphate, calcium phosphate, calcium pyrophosphate, potassium dihydrogen phosphate, dipotassium hydrogen phosphate, sodium acid pyrophosphate, sodium dihydrogen phosphate, disodium hydrogen phosphate, sodium phosphate, and sodium pyrophosphate.

Preferably, the carbonate salt may be at least one selected from the group consisting of sodium carbonate, potassium carbonate, sodium bicarbonate, and potassium bicarbonate.

Preferably, the phytone may be soya peptone.

Preferably, the defoamer may be at least one selected from lauric acid, palmitic acid, a glyceride of a fatty acid, a calcium soap, an aluminum soap, and a magnesium soap of stearic acid, a calcium soap, an aluminum soap, and a magnesium soap of palmitic acid, and a fatty alcohol.

A preparation method of an immobilized β-galactosidase is provided, including the following steps:
mixing an ion exchange resin with a β-galactosidase liquid to allow a reaction to proceed to obtain the immobilized β-galactosidase.

Preferably, the ion exchange resin may include a tertiary amine group.

Preferably, the ion exchange resin may be a styrene series weakly alkaline anion exchange resin; more preferably, the ion exchange resin may be a macroporous styrene series weakly alkaline anion exchange resin having tertiary amine functional groups.

Preferably, the ion exchange resin and the β-galactosidase liquid may be at a ratio of 1 g: (70-140) U.

Preferably, the reaction may be conducted at a temperature of 6-35°C; more preferably, the reaction may be conducted at a temperature of 8-25°C; still more preferably, the reaction may be conducted at a temperature of 8-20°C.

Preferably, the reaction may last for 16-24 h.

Preferably, before use, the ion exchange resin should be soaked in water for 2-5 h to obtain an activated resin. This step is intended to keep the ion exchange resin moist and prevent the ion exchange resin from a structural change due to over-drying.

Preferably, the water may be deionized water.

Preferably, the reaction may be carried out with a conductivity of 4-20 ms/cm (that is, the conductivity of the β-galactosidase liquid may be adjusted to 4-20 ms/cm).

Preferably, a preparation method of an immobilized β-galactosidase may be provided, including the following steps:
soaking an ion exchange resin in deionized water for 2-5 h to obtain an activated ion exchange resin, mixing the ion exchange resin with a β-galactosidase liquid at a ratio of 1 g: (70-140) U, stirring with a conductivity of 4-20 ms/cm, and reacting for 16-24 h to prepare the immobilized β-galactosidase.

A preparation method of GOSs is provided, including the following steps:
mixing an immobilized β-galactosidase with lactose, stirring and reacting to obtain GOSs.

Preferably, the immobilized β-galactosidase and the lactose may be at a ratio of 3,000 U -25,000 U of the immobilized β-galactosidase per kg of the lactose.

Preferably, the lactose may be a lactose solution, and the mass concentration of the lactose solution may be 35-55%.

Preferably, the reaction may be conducted at a temperature of 45-60°C for 6-35 h.

Preferably, the lactose may be at least one selected from the group consisting of food-grade lactose, pharmaceutical-grade lactose, and filtered whey powder (the food-grade lactose, pharmaceutical-grade lactose, or filtered whey powder may be conventional commercial products).

Compared with the food-grade lactose and the pharmaceutical-grade lactose, the filtered whey powder has higher salt content, and contains more nutrients such as B vitamins and proteins.

Compared with the prior art, the present disclosure has the following beneficial effects:
(1) The biggest advantage of the present disclosure is to solve the problems associated with the process and cost of large-scale production of GOSs. Due to the use of the fermentation medium of the present disclosure, the fermentation time to produce β-galactosidase in the present disclosure is shortened, and the production cycle is shortened. Moreover, with a simple fermentation mode, no additional feeding is required. Although additional feeding at the late fermentation stage in the prior art is often conducive to an increase in enzyme activity, compounding in batches increases the burden on personnel and equipment investment, and there is a greater hidden danger of contamination.
(2) The strain used for producing β-galactosidase of the present disclosure is non-genetically modified, and is safe and reliable. The enzyme liquid produced according to the present disclosure has relatively high specific activity and may reduce the amount of resin used during the immobilization of the enzyme. After immobilization, the enzyme shows a higher activity in converting lactose into GOSs.
(3) Crosslinking agents and other chemical reagents are not needed in the preparation of the immobilized β-galactosidase of the present disclosure, thus preventing any potential impact on food safety due to the presence of residual crosslinking agent; moreover, the immobilized enzyme prepared has excellent stability and can be used in more than 10 cycles or can be used continuously for 264 h or longer. The most direct impact on the preparation of GOSs is the reduction of the cost of enzymes.

### BRIEF DESCRIPTION OF THE DRAWINGS

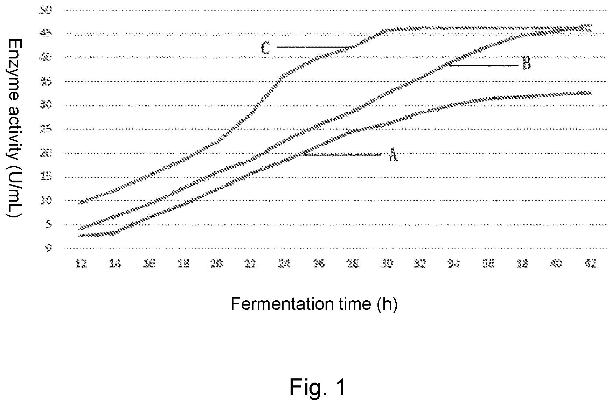

FIG. 1 illustrates a comparison of the enzyme activity and fermentation time of fermentation broths in fermentors A, B, and C in Example 6.

### DETAILED DESCRIPTION

In order to enable those skilled in the art to understand the technical solutions of the present disclosure more clearly, the following examples are provided for description now. It should be pointed out that the following examples do not limit the scope of protection claimed by the present disclosure.

Unless otherwise specified, all raw materials, reagents, or devices used in the following examples are commercially available or can be obtained by known methods.

Several concepts described in the present disclosure are explained below.

The definition of β-galactosidase activity: 1 mL of β-galactosidase liquid is added into 1 mL of 0.1 M. phosphate buffer solution (PBS, pH 6.0) containing 4 mg/mL o-nitrophenyl β-D-galactopyranoside (ONPG) to react at 50°C for 10 min, and the reaction is terminated with 2 mL of 10% (mass fraction) sodium carbonate solution; the amount of hydrolyzed ONPG is calculated by determining the content of o-nitrophenol in the product. Under these conditions, the amount of enzyme required to hydrolyze 1 µM ONPG per minute is defined as one active unit (U). The ONPG can be hydrolyzed into galactose and yellow o-nitrophenol by β-galactosidase.Therefore, the β-galactosidase activity can be detected by the color change of a culture medium.

Standard for determination of GOS content: Refer to the AOAC Official Method 2001.02 described in the Determination of trans-Galacto-oligosaccharides (TGOS) in Selected Food Products*.*

### Example 1: Preparation of β-galactosidase

A preparation method of β-galactosidase was provided, including the following steps:
step 1, *B. circulans* (purchased from the ATCC, No. 31382) was seeded into an LB medium, cultured at 37°C for 18 h, and activated; the cultured and activated *B. circulans* was added to an Erlenmeyer flask containing 500 mL of LB medium, cultured with shaking at 37°C and 180 rpm for 6 h, and seeded into a seed tank for culture to obtain a seed culture broth;
step 2, the seed culture broth prepared in step 1 was introduced to a 200 L fermentor containing 120 L of fermentation medium for fermentation, and the pH in the fermentor was adjusted to 6.64 with 10% (mass fraction) sodium hydroxide solution; the seed cultured broth was stirred at 37 ± 0.5°C and 180 rpm, and fermented at an aeration rate of 120 L/min for 8 h; after 8 h, the pH was adjusted to 7.45 ± 0.5 with hydrochloric acid; after 12 h, samples were taken every 2 h to assess enzyme activity until the enzyme activity did not rise any more during fermentation; 112 L of fermentation broth of *B. circulans* with an enzyme activity of 31.2 U/mL was obtained after fermenting for 42 h;
step 3, the fermentation broth of *B. circulans* obtained in step 2 was filtered through a microfiltration membrane to separate an enzyme liquid from cells at 6°C, and the enzyme liquid was concentrated with a 50 KD membrane to obtain 31.8 L of β-galactosidase liquid with an enzyme activity of 101.7 U/mL;
in step 2, the fermentation medium included 1,200 g of lactose, 960 g of galactose, 1,920 g of soya peptone, 486 g of dried corn steep liquor powder, 312 g of yeast extract, 312 g of diammonium hydrogen phosphate, 156 g of sodium carbonate, 100 g of defoamer, and water.

### Example 2: Preparation of β-galactosidase

The difference between Examples 2 and 1 was that *B. circulans* CCTCC NO: M2015424 was used instead of *B. circulans* (purchased from the ATCC, No. 31382) in step 1 of Example 2, and 115 L of fermentation broth of *B. circulans* with an enzyme activity of 46.3 U/mL was obtained after 30 hours of fermentation; 46.0 L of enzyme liquid with an enzyme activity of 107.6 U/mL was obtained after microfiltration to remove cells and concentration with a 50 KD membrane.

### Example 3: Preparation of immobilized β-galactosidase

### 1. Refining of crude enzyme

6.9 kg of ammonium sulfate was slowly introduced added to 46 L of the enzyme liquid obtained after concentration in Example 2. After the ammonium sulfate was completely dissolved, the enzyme liquid was allowed to stand at 6°C for 1 h, and washed with a 50 KD membrane to obtain 46.8 L of refined β-galactosidase liquid with an enzyme activity of 102.1 U/mL (that is, the β-galactosidase activity was 102.1 U per 1 mL of β-galactosidase liquid) and a conductivity of 7.84 ms/cm.

### 2. Preparation of immobilized β-galactosidase

A preparation method of immobilized β-galactosidase was provided, including the following steps:
42 Kg of macroporous styrene series weakly alkaline anion exchange resin A103s (the resin was provided by Purolite (China) Co., Ltd., with a resin pore size of 30-50 nm) was soaked in deionized water for 4 h and filtered to obtain activated styrene series weakly alkaline anion exchange resin A103s; the macroporous styrene series weakly alkaline anion exchange resin A103s was mixed with 46.8 L of refined β-galactosidase liquid with an enzyme activity of 102.1 U/mL and a conductivity of 7.84 ms/cm, and reacted under stirring at 12°C and 30 rpm for 16 h. After washing with deionized water, 41.1 kg of immobilized β-galactosidase with an enzyme activity of 111.6 U/g (that is, the β-galactosidase activity was 111.6 U per g of the immobilized enzyme) was obtained.

### Example 4: Preparation of GOSs using the immobilized β-galactosidase

A preparation method of GOSs was provided, including the following steps:
800 Kg of 50 wt% lactose solution (with a lactose content of 400 kg) and 25 kg of the immobilized β-galactosidase prepared in Example 3 (that is, corresponding to an enzyme activity of 6.98 U per g of lactose) were successively added to a 1,000 L reaction tank. After reacting at 50°C and 80 rpm for 14 h, reaction products were separated by a 100 mesh filter screen to obtain an immobilized enzyme and a syrup. The syrup was filtered and concentrated by a vacuum concentrator to obtain a 75.8 Brix syrup with 58.9 wt% GOSs, and the immobilized enzyme could be put into the reaction tank again to allow a reaction to proceed.

### Example 5: Preparation of GOSs using the immobilized β-galactosidase

A preparation method of GOSs was provided, including the following steps:
800 Kg of 50 wt% filtered whey powder (by mass fraction, in the filtered whey powder, lactose, protein, and conductivity ash accounted for 95.74%, 1.26%, and 0.21% on a dry basis, respectively) solution (with a filtered whey powder of 400 kg) and 25 kg of the immobilized β-galactosidase prepared in Example 3 (that is, corresponding to an enzyme activity of 4.40 U per g of lactose) were successively added into a 1,000 L reaction tank. After reacting at 50°C and 80 rpm for 15 h, the products were separated by a 100 mesh filter screen to obtain an immobilized enzyme and a syrup. The syrup was separated from the immobilized enzyme by filtration, and was concentrated by a vacuum concentrator to obtain a 75.6 Brix syrup with 57.8 wt% GOSs. The immobilized enzyme could be introduced into the reaction tank again to allow a reaction to proceed.

### Example 6: Effect of different fermentation conditions on the ability of B. circulans to produce β-galactosidase

Based on the steps in Example 1, the following three fermentation conditions were set upin fermentors A, B, and C. Each fermentor was 5 L; the fermentation broth (which refers to a mixture of seed culture broth and fermentation medium, including fermentation products) was 2,500 mL. Compared with Example 1, the major changes of the fermentation conditions in the three fermentors included the components of the fermentation medium, fermentation pH in the fermentor in step 2, and fermentation strains. Any fermentation conditions not specifically described were the same as those in Example 1. Specifically:
Fermentor A: The fermentation medium included 25 g of lactose, 20 g of galactose, 40 g of soya peptone, 10 g of dried corn steep liquor powder, 6.5 g of yeast extract, 6.5 g of diammonium hydrogen phosphate, 3.25 g of sodium carbonate, 1.5 g of defoamer, and water. The strain was *B*. *circulans* ATCC No. 31382. The condition for fermentation in the fermentor in step 2 was fermentation at a pH of 7.4.
Fermentor B: The fermentation medium included 12.5 g of lactose, 20 g of galactose, 40 g of soya peptone, 10 g of dried corn steep liquor powder, 6.5 g of yeast extract, 6.5 g of diammonium hydrogen phosphate, 3.25 g of sodium carbonate, 1.5 g of defoamer, and water. The strain was *B. circulans* ATCC No. 31382. The condition for fermentation in the fermentor in step 2 was fermentation at a pH of 7.4. During the fermentation in step 2, 125 g of 10 wt% lactose solution was fed.
Fermentor C: The fermentation medium included 25 g of lactose, 20 g of galactose, 40 g of soya peptone, 10 g of dried corn steep liquor powder, 6.5 g of yeast extract, 6.5 g of diammonium hydrogen phosphate, 3.25 g of sodium carbonate, 1.5 g of defoamer, and water. The strain was *B*. *circulans* CCTCC NO: M 2015424; the fermentation conditions in the fermentor in step 2 were fermentation at a pH of 6.75 ± 0.05 with 0-8 h and pH 7.4 after 8 h.

After fermentation in the fermentor for 12 h in step 2, samples were taken every 2 h to assess enzyme activity. The results are shown in FIG. 1. FIG. 1 illustrates a comparison of the relationships between fermentation time and enzyme activities of fermentation broths in fermentors A, B, and C in Example 6. Curve A represents a relationship between fermentation time and the enzyme activity of the fermentation broth in fermentor A, curve B represents the relationship between fermentation time and the enzyme activity of the fermentation broth in fermentor B, and curve C represents the relationship between fermentation time and the enzyme activity of the fermentation broth in fermentor C.

In FIG. 1, curve C corresponding to fermentor C shows that in fermentor C, the fermentation rate is faster, the enzyme activity is higher in the early stage, and the enzyme activity in the fermentation broth has reached 40 U/mL at 26 h and 45.88 U/mL at 42 h, which is close to the enzyme activity of 46.80 U/mL in fermentor B at 42 h. That is, without additional feeding, a higher enzyme activity level can be reached in 28-30 h for fermentor C. The production time is shortened by 8-12 h compared to that of fermentor B. As a result, the production cycle can be effectively shortened and energy consumption during fermentation can be reduced. In addition, batch sterilization and batch are not required, eliminating potential dangers of bacterial contamination caused by feeding, and simplifying the production process. It can be seen from curves A and C that the fermentation is slow and the enzyme activity is low in fermentor A. Hence, the selection of fermentation strains and the control of pH during the fermentation also have important impacts on the enzyme activity in the fermentation broth.

### Example 7: Effects of different fermentation conditions on the immobilization of β-galactosidase produced by B. circulans

The enzyme liquids of fermentors A, B, and C in Example 6 were first separated by microfiltration to determine their protein contents. Their specific enzyme activities (enzyme activity divided by mg of protein) were calculated. To each of the enzyme liquids, water-activated macroporous styrene series weakly alkaline anion exchange resin A103s was added at an amount of 200 U/g resin (the amount of enzyme added was greater than the adsorption capacity of the resin, that is, allowing saturated adsorption of the resin) and stirred at 12°C and 30 rpm for 20h to allow an adsorption reaction to take place; then, the enzyme activity in per unit of resin was calculated. Subsequently, a 50% lactose solution was added at an amount of 5 U per g of lactose and converted for 24 h under the reaction conditions in Example 4. The GOS content was determined. The results are shown in Table 1 below:

**Table 1:**

| Fermentor No. | Specific enzyme activity (U/mg of protein) | Enzyme activity U per g of resin (U/g of resin) | GOS content 24 h after conversion (%) |
|---|---|---|---|
| Fermentor A | 35.24 | 86.15 | 53.72 |
| Fermentor B | 43.78 | 101.24 | 56.83 |
| Fermentor C | 51.36 | 119.32 | 57.21 |

The specific enzyme activity directly reflects the purity of the enzyme. From the results in Table 1, it can be seen that different strains and fermentation conditions (including fermentation time) affect the purity of the enzyme. Higher purity of the enzyme results in fewer carriers (resin) required during the immobilization of the enzyme, thus lowering production costs; moreover, the enzyme shows better ability in converting lactose to GOSs after immobilization.

### Example 8: Effects of the preparation conditions of the immobilized β-galactosidase on the performance of β-galactosidase

### 1. Effect of crosslinking agent on immobilized β-galactosidase

3.3 g of macroporous styrene series weakly alkaline anion exchange resin A103s washed with deionized water was respectively added to two 250 mL Erlenmeyer flasks (labelled as Erlenmeyer flasks A and B); deionized water and 0.5 mL of 30 wt% glutaraldehyde were added to Erlenmeyer flask A such that the mass concentration of glutaraldehyde in Erlenmeyer flask A was about 0.15%, and a reaction was conducted at 25°C for 12 h; 0.5 mL of deionized water was added to Erlenmeyer flask B; subsequently, the β-galactosidase liquid prepared in Example 1 was separately added to Erlenmeyer flasks A and B at an amount of 110 U/g of resin, followed by reacting at 10°C for 16 h to prepare immobilized β-galactosidase.

The immobilized enzymes prepared in Erlenmeyer flasks A and B were used to continuously covert 8 batches in a shake flask an amount of 10 U/g of lactose; that is, the immobilized enzymes were used in 8 cycles, and the conversion time for each cycle was 24 h. The 8^{th} GOS conversion rate was 51.96% for the immobilized enzyme prepared in Erlenmeyer flask A and 55.32% for the immobilized enzyme prepared in Erlenmeyer flask B, respectively. It can be seen that the immobilized enzyme prepared without using glutaraldehyde as a crosslinking agent shows a more stable conversion performance.

In the 8^{th} cycle of the immobilized enzyme prepared without a crosslinking agent, the amount of GOS produced still reached 55% (namely, the GOS content in the product was 55% with an identical amount of reactants and identical reaction conditions); this is only a slight decrease compared with 57% of the first batch. In Patent Application No. CN 201480049114.3, after 8 cycles of reactions, the GOS content decreased from 69.00% to 48.93%. The catalytic performance of the immobilized β-galactosidase produced by the method of the present disclosure is more stable. Calculating in terms of a 24-hour conversion time for each cycle, after continuous conversion for 264 h (namely 11 cycles), the GOS content still reached more than 54%.

### 2. Effect of conductivity condition on the immobilized β-galactosidase

4 mL of β-galactosidase liquid with an enzyme activity of 98.72 U/mL was respectively added to two 50 mL Erlenmeyer flasks C and D; the conductivity in Erlenmeyer flask C was adjusted to 10.06 ms/cm with 1% (mass fraction) ammonium sulfate solution; the conductivity in Erlenmeyer flask D was not adjusted (the conductivity was detected as 2.03 ms/cm); 3.3 g of activated macroporous styrene series weakly alkaline anion exchange resin A103s was respectively added to Erlenmeyer flasks C and D. The adsorption reaction was performed on a shaker at 10°C, and the shaker shakes slightly so that the macroporous styrene series weakly alkaline anion exchange resin A103s could contact evenly with the β-galactosidase liquid; immobilized enzymes C and D were obtained 16 h after reaction. The enzyme activity of the residual liquid in each Erlenmeyer flask was determined. The enzyme activity of the residual liquid was 1.06 U/mL in Erlenmeyer flask C and 0.45 U/mL in Erlenmeyer flask D.

The immobilized enzymes C and D prepared were reacted with lactose, and the conversion abilities of these enzymes were examined by continuous flask shaking. The immobilized enzyme C was allowed to continuously convert for 5 cycles (120 h), the GOS content of the product after the 5^{th} cycle was 55.84%. The immobilized enzyme D was allowed to convert for 5 times (120 h), the GOS content of the product at the 5^{th} cycle was 46.28%. It can be seen that the conductivity environment during the production of the immobilized enzyme has a significant effect on the service life of the immobilized enzyme.

## Claims

1. A preparation method of β-galactosidase, comprising the following steps:
step 1, seeding *Bacillus circulans* into a Luria-Bertani (LB) medium for culture and activation and then into a seed tank for culture to obtain a seed culture broth;
step 2, introducing the seed culture broth obtained in step 1 into a fermentor containing a fermentation medium for fermentation to obtain a fermentation broth of *Bacillus circulans;* and
step 3, filtering or centrifuging the fermentation broth of *Bacillus circulans* obtained in step 2 to remove cells to obtain a β-galactosidase liquid;
wherein the fermentation medium comprises lactose, galactose, phytone, corn meal, yeast extract, a phosphate salt, a carbonate salt, and water.

2. The preparation method according to claim 1, wherein the *Bacillus circulans* used in step 1 is *Bacillus circulans* CCTCC NO: M 2015424, deposited in the China Center for Type Culture Collection (CCTCC).

3. The preparation method according to claim 1, wherein the fermentation medium in step 2 comprises 5-30 g/L lactose, 5-10 g/L galactose, 10-30 g/L phytone, 2-5g/L dried corn steep liquor powder, 2-4 g/L yeast extract, 2-4 g/L the phosphate salt, 1-2 g/L the carbonate salt, the balance being water.

4. The preparation method according to claim 1, wherein the fermentation in step 2 is conducted at a pH of 5.5-8.0 for 24-35 h.

5. The preparation method according to claim 4, wherein the fermentation in step 2 is conducted at a pH of 6.5-7.0 for 0-8 h and a pH of 7.2-7.6 after 8 h.

6. A preparation method of immobilized β-galactosidase, comprising the following steps:
mixing an ion exchange resin with the β-galactosidase liquid prepared by the preparation method according to any one of claims 1 to 5 to allow a reaction to proceed to obtain the immobilized β-galactosidase.

7. The preparation method according to claim 6, wherein the ion exchange resin comprises a tertiary amine group; and the ion exchange resin and the β-galactosidase liquid are at a ratio of 1 g: (70-140) U.

8. The preparation method according to claim 6, wherein the reaction is carried out with a conductivity of 4-20 ms/cm.

9. A preparation method of a galacto-oligosaccharide, comprising the following steps:
mixing the immobilized β-galactosidase prepared by the preparation method according to any one of claims 6 to 8 with lactose, stirring and reacting to obtain the galacto-oligosaccharide.

10. The preparation method according to claim 9, wherein the immobilized β-galactosidase and the lactose are at a ratio of 3,000 U -25,000 U of the immobilized β-galactosidase per kg of the lactose; the lactose is at least one selected from the group consisting of food-grade lactose, pharmaceutical-grade lactose, and filtered whey powder.
